# EUROPEAN PATENT SPECIFICATION

(11) **EP 1 652 903 B1**
(45) Date of publication and mention of the grant of the patent: **08.09.2010**
(21) Application number: 05109397.9
(22) Date of filing: 10.10.2005
(51) Int. Cl.: C09K 15/30, C07D 275/02, A01N 43/80

(54) **Isothiazolone containing preservative having enhanced stability**
Isothazolon enthaltendes Konservierungsmittel mit verbesserter Stabilität
Agent de conservation contenant de l'isothiazolone et présentant une stabilité améliorée

(30) Priority: 02.11.2004 DE 102004052878
(43) Date of publication of application: 03.05.2006
(73) Proprietor: AIR LIQUIDE SANTE (INTERNATIONAL), 75007 Paris (FR); Schulke & Mayr, 22851 Norderstedt (DE)
(72) Inventor: Weber, Klaus, 20149 Hamburg (DE); Beilfuss, Wolfgang, 22339 Hamburg (DE); Gradtke, Ralf, 25436 Tornesch (DE)
(74) Representative: Conan, Philippe Claude

(56) References cited:
- EP-A- 1 537 782
- EP-B- 0 982 993
- GB-A- 2 011 790
- US-A1- 2004 181 001

## Description

The present invention relates to a preservative which comprises an isothiazolone and to the condensation product of a mixture which comprises glycol, urea and formaldehyde. The invention further relates to the use of the preservative in technical products, to the technical product and to the use of the condensation product for stabilizing isothiazolones. The inventive preservative is free from 5-chloro-2-methylisothiazolone and is free from 2-mercaptopyridine N-oxide and derivatives thereof.

Isothiazolin-3-ones (isothiazolones) are known as active bactericidal and fungicidal compounds, for example in cooling lubricants for metalworking. However, certain components in metalworking fluids and a high pH have a tendency to destroy the isothiazolones and thus eliminate their microbiological activity. This is the case, in particular when the metalworking fluids are present in concentrated form. Therefore there has been no lack of attempts to propose stabilizers for isothiazolones.

EP 0 425 143 A, US 4,150,026 A and US 4,241,214 A disclose that metal salt complexes of isothiazolones can be used owing to their enhanced thermal stability, while the biological activity of the isothiazolones is retained. EP 0 315 464 A discloses orthoesters, and EP 0 342 852 A discloses epoxides as stabilizers.

US-A-4,129,448 and US-A-4,165,318 propose to use, as stabilizer for isothiazolones, formaldehyde or a compound which releases formaldehyde under basic conditions. As formaldehyde-releasing compounds, various quaternary ammonium chlorides are proposed. Apart from the fact that these substances are comparatively expensive chemicals, chlorine-containing preservatives are frequently undesirable. Isothiazolone-containing preservative concentrates are not disclosed.

It is further known to use sulphur compounds for stabilizing isothiazolones (EP 0 777 966 A, GB 2 208 474 A, US-A-5,725,806, GB 2 230 190 A and US 5,210,094 A1, US 5,210,094 A). DE 195 34 532 A, in addition to a sulphur-containing stabilizer, proposes the use of a solubilizer. EP 0 530 986 A2 proposes stabilizing isothiazolones by adduct formation with nitrogen-containing, sulphur-free compounds, for example pyridine N-oxide, 2-pyrrolidone, s-triazine or dimethyl oxime.

The stability of isothiazolones in combination with N-formals is also unsatisfactory. For instance storage, transport or use at elevated temperatures lead to the breakdown of isothiazolone and thus to a preservative no longer having sufficient microbicidal activity. Therefore, DE 199 61 621 A1 prescribes the presence of 2-mercaptopyridine N-oxide or salts thereof for stabilizing isothiazolones in the event of an N-formal content.

DE 28 00 766 A discloses a preservative which, in addition to 5-chloro-2-methylisothiazolone, comprises a mixture of 20-30% of dimethylolurea, 50-75% of one or more adducts of 2 mol of formaldehyde and aliphatic glycols having 2-8 carbon atoms and/or monoalkyl ethers of these glycols having 1-6 carbon atoms in the ether radical. These adducts are O-formals, the presence of which, because of elevated formaldehyde emissions and odour nuisances at elevated temperatures, is disadvantageous.

Furthermore, a combination of Kathon 886 (that is to say 5-chloro-2-methylisothiazolone and 2-methylisothiazolone in a ratio of 3:1 in about 14% strength aqueous solution) with the condensation product of glycol, urea and formaldehyde is known as a commercial product. The combination, however, has very limited stability at elevated temperatures (for example above 30°C) and is sensitive to pH effects, and must therefore be stabilized with compounds such as 2,2'-dithiobis(pyridine N-oxide) (Pyrion disulfide).

The document EP-A-1,537,782, which was published after the date of filing of the present patent application, relates to a biocide composition comprising one or more isothiazolones and a formaldehyde releasing composition comprising urea, formaldehyde and paraformaldehyde and a glycol and its use for treating acqueous systems.

The document EP-A-0,982,993 discloses compositions or preservatives for use industrial products, and industrial products showing increased stability. For example, this document discloses a composition containing 3,3'-methylenebis (5-methylloxyalidine) with n-octylisothiazolone and 1,2-propylene glycol hemiformal.

The document US2004/181001 relates to a synthetic stucco composition comprising preservative solution of ureaformaldehyde condensation products and isothiazolinone derivatives.

The object underlying the present invention was thus to provide substances which
- stabilize isothiazolones and isothiazolone-containing mixtures during storage, during transport and in use, even at elevated temperatures, without discoloration or odour nuisance occurring,
- have advantages with respect to the official labelling regulations,
- stabilize isothiazolones even in the form of a concentrate,
- support isothiazolones in their microbicidal activity and
- are inexpensive.

It has now surprisingly been found that this object is achieved by the condensation product of a mixture which comprises glycol, urea and formaldehyde.

The invention thus relates to a preservative which comprises
a) isothiazolone and
b) the condensation product of a mixture which comprises 24 to 44% by weight of glycol, 17 to 32% by weight of urea and 33 to 60% by weight of formaldehyde,
the preservative being free from 5-chloro-2-methylisothiazolone and free from 2-mercaptopyridine N-oxide and derivatives thereof.

The invention is based, inter alia, on the fact that it has been found that a particularly stable isothiazolone-containing preservative can be obtained, even without addition of 2-mercaptopyridine N-oxide and derivatives thereof (for example without the sodium salt, Pyrion-Na, and without the dithioether, Pyrion disulfide), if, in combination with the isothiazolone (different from 5-chloro-2-methylisothiazolone), use is made of the condensation product of a mixture which comprises glycol, urea and formaldehyde.

### Isothiazolone

Isothiazolones used according to the invention are preferably selected from 2-n-octylisothiazolone, for example the pure active compound (Kathon 893T) or the 45% strength solution in 1,2-propylene glycol (Kathon 893), 2-methylisothiazolone, 4,5-dichloro-2-n-octylisothiazolone, 2-n-butylisothiazolone and benzoisothiazolone, 2-n-octylisothiazolone being particularly preferred.

In a preferred embodiment, the preservative is present as concentrate and then comprises 0.25-5% by weight of isothiazolone, preferably 0.5-3% by weight, for example 0.7 to 1.4% by weight, for instance 1.1% by weight of isothiazolone.

### Condensation product

According to the invention, as stabilizer, use is made of the condensation product of a mixture which comprises glycol, urea and formaldehyde. It has surprisingly been found that these condensation products yield extraordinarily stable and in particular thermally stable compositions, that is to say concentrates and preserved technical products, having a content of isothiazolone (different from 5-chloro-2-methylisothiazolone). This increase in stability is demonstrated in a particularly noticeable manner in storage under thermal stress, as can occur during transport and storage, or during processing. The advantage is observed, not only in the concentrate, but also in the preserved technical product, with, surprisingly, no presence of 2-mercaptopyridine N-oxide or derivative thereof being necessary for the stabilization.

In a preferred embodiment, the preservative is present as concentrate and then comprises 99.75-95% by weight of the condensation product, preferably 99.5-97% by weight, for example 99.3-98.6% by weight, for instance 98.9% by weight of condensation product.

In a preferred embodiment, the glycol is monoethylene glycol. In a further preferred embodiment, the mixture further comprises a small amount of potassium carbonate. In a particularly preferred embodiment, though the mixture is free from butyl diglycol, such preservatives have a particularly good stability.

In a particularly preferred embodiment, the mixture processed to give the condensation product comprises monoethylene glycol, a small amount of potassium carbonate, urea and formaldehyde (used as paraformaldehyde). Mixtures are preferred which consist of about 34% by weight of monoethylene glycol, a small amount of potassium carbonate, about 14% by weight of urea and about 52% by weight of paraformaldehyde (91% strength).

Preferred mixtures comprise glycol, urea and formaldehyde in the following amounts (% by weight).

| | Preferred | Preferred | In particular |
|---|---|---|---|
| Glycol (for example monoethylene glycol) | 24-44 | 29-39 | 32-36 |
| Urea | 17-32 | 20-28 | 23-25.5 |
| Formaldehyde | 33-60 | 40-55 | 45-50 |

The constituents can be added to the mixture in various ways. In a variant A, a mixture comprising a ureaformaldehyde adduct (for example dimethylol urea) and the reaction product of glycol and formaldehyde is prepared. In a variant B, a mixture comprising a ureaformaldehyde adduct (for example dimethylol urea) and an alkylene glycol formal is prepared. In a variant C, a mixture comprising urea, formaldehyde and the reaction product of glycol with formaldehyde is prepared. In the particularly preferred variant D, a mixture is prepared which comprises formaldehyde, urea, ethylene glycol and if appropriate a small amount of potassium carbonate. In a variant E, a mixture is prepared which comprises the reaction product of formaldehyde with glycol with addition of urea.

After preparation of the mixture, this is processed to give the condensation product. The condensation is performed at a temperature of at least about 40°C, preferably at least about 50°C, more preferably 70°C to 105°C, in particular 90°C to 100°C, a condensation temperature of 95 to 100°C being particularly preferred. After the end of the condensation reaction, preferably no water is taken off. In a preferred embodiment, the condensation of the mixture is performed at 95°C for a period of 3 hours.

In a preferred embodiment, the preservative is present as concentrate and contains no added organic solvent, apart from a small amount of, for example, less than 3% by weight, preferably less than 2% by weight, possibly introduced together with the isothiazolone. Preferred inventive concentrates have a water content of less than 45% by weight, preferably 2 to 30% by weight, more preferably 5 to 15% by weight, for instance 10% by weight.

Inventive preservatives which are present in the form of a concentrate preferably comprise
a) 0.25-5% by weight, preferably 0.5-3% by weight, for example about 1.1% by weight, of isothiazolone and
b) 99.75-95% by weight, preferably 99.5-97% by weight, for example about 98.9% by weight, of the condensation product.

Concentrates are preferred which essentially consist of these two components, with, if appropriate, a small amount of fragrance being able to be added.

The invention further relates to the use of the inventive composition for preserving technical products, in particular crop protection agents, agents for treating seed material, technical preservatives, in particular container preservatives, cooling lubricants, fuel additives, disinfectants, in particular low-foam disinfectants, agents for controlling incised wounds, parasites and plants, agents for treating pruning wounds, film preservatives for the outdoor, and in particular the indoor, sector, disinfectants in areas where increased fungal attack is to be expected, agents for preserving concrete additives, technical preservatives for tropical zones and wood preservatives. A preferred amount of the concentrate in the technical products is 0.01-1% by weight, more preferably 0.05-0.5% by weight, in particular 0.1-0.3% by weight, for instance 0.15% by weight.

The invention further relates to the use of the condensation product of a mixture which comprises glycol, urea and formaldehyde for stabilizing isothiazolones (different from 5-chloro-2-methylisothiazolone) without addition of 2-mercaptopyridine N-oxide and derivatives thereof.

The advantages of the present invention are demonstrated, in particular, by the examples hereinafter.

### Examples

The content of 2-n-octylisothiazolone was determined by HPLC, in which each 0.5 g of sample was weighed into a 50 ml measuring flask and made up with 50% strength H₃PO₄ (0.1 strength)/50% acetonitrile. The solution was then injected into the measuring apparatus. As a reference sample, 100 mg of Kathon 893 was weighed into a 100 ml measuring flask and made up with 85% strength H₃PO₄ (0.1% strength)/50% acetonitrile, then injected into the measuring apparatus. The measuring apparatus used was an HPLC combination of a pump (Waters 600), a detector (Waters PDA 996), an injection system (Waters Autosampler WISP 717), a column (Nucleosil 100, C 18.5, 10 pm, 100 x 4 mm i.d. using the eluent A = H₃PO₄ (0.1% strength (g/g)) and B = acetonitrile. Elution was performed using 90% A/10% B in 10 min to 20% A, 80% B, then in 7 min to 10% A, 90% B, then washing and conditioning the column. Flow rate 1.0 ml/min, wavelength: 273 nm, injection volume 10 µl.

Concentrates were prepared using the following constituents and tested for their storage stability:
- Condensation product A was prepared by heating at 95°C for 3 hours a mixture of 24.82% by weight of butyl diglycol, 21.15% by weight of monoethylene glycol, 1.10% by weight of potassium carbonate, 12.00% by weight of urea and 40.93% by weight of paraformaldehyde (90% pure).
- Condensation product B was prepared by heating at 95°C for 3 hours a mixture of 33.76% by weight of monoethylene glycol, 0.20% by weight of potassium carbonate, 24.21% by weight of urea and 51.83% by weight of paraformaldehyde (91% pure).
- Methylenebismethyloxazolidine, trimethyltriazinetriethanol and triazinetriethanol are commercially conventional N-formals.
- OIT = 2-n-octylisothiazolone as Kathon 893 (figures in % by weight).

| | A | B | C (comparison) | D (comparison) | E (comparison) |
|---|---|---|---|---|---|
| OIT | 2.5% | 2.5% | 2.5% | 2.5% | 2.5% |
| Condensation product A | 97.5% | | | | |
| Condensation product B | | 97.5% | | | |
| Methylenebismethyloxazolidine | | | 97.5% | | |
| Trimethyltriazinetriethanol | | | | 97.5% | |
| Triazinetriethanol | | | | | 97.5% |
| After mixing | clear colourless solution | clear colourless solution | clear colourless solution | clear colourless solution | clear yellowish solution |
| Storage at room temperature OIT content after 2 days | 1.17 | 1.15% | 1.18% | 0.07% | 0.12% |
| Appearance after 5 days | clear colourless solution | clear colourless solution | clear colourless solution | turbid yellow liquid | turbid yellow liquid |
| Storage at 40°C, | | | | | |
| - OIT content after 1 month | 1.16% | 1.19% | 1.19% | <0.01% | <0.01% |
| - Appearance after 1 month | clear colourless solution | clear colourless solution | clear colourless solution | clear orange solution | clear dark-yellow solution |
| Storage at 40°C, | | | | | |
| - OIT content after 2 months | 1.08% | 1.12% | 1.12% | <0.01% | <0.01% |
| - Appearance after 2 months | clear colourless solution | clear colourless solution | clear colourless solution | clear orange solution | clear orange-yellow solution |
| Storage at 40°C, | | | | | |
| - OIT content after 3 months | 1.09% | 1.14% | 1.13% | <0.01% | <0.01% |
| - Appearance after 3 months | clear colourless solution | clear colourless solution | clear colourless solution | clear orange solution | clear orange-yellow solution |
| Storage at 60°C, | | | | | |
| - OIT content after 1 month | 0.57% | 1.17% | 0.01% | <0.01% | <0.01% |
| - Appearance after 1 month | clear yellow solution | clear colourless solution | clear yellow solution | clear red solution | clear red solution |
| Storage at 60°C, | | | | | |
| - OIT content after 2 months | <0.01% | 1.08% | <0.01% | <0.01% | <0.01% |
| - Appearance after 2 months | clear yellow solution | clear colourless solution | clear yellow solution | clear red solution | clear red solution |
| Storage at 60°C, | | | | | |
| - OIT content after 3 months | <0.01% | 1.05% | <0.01% | <0.01% | <0.01% |
| - Appearance after 3 months | clear orange-yellow solution | clear colourless solution | clear yellow solution | clear red solution | clear red solution |

These results verify that N-formals cannot stabilize isothiazolones even at room temperature (according to the teaching of DE 199 61 621, the addition of a stabilizer such as Pyrion-sodium is necessary). In contrast, 2-n-octylisothiazolone is made very storage stable by inventive condensation products even under thermal stress, the stabilization using condensation product B (mixture without butyl diglycol) being better than using condensation product A (see the results for storage at 60°C).

## Claims

1. Preservative which comprises
a) isothiazolone and
b) the condensation product of a mixture which comprises 24 to 44% by weight of glycol, 17 to 32% by weight of urea and 33 to 60% by weight of formaldehyde,
the preservative being free from 5-chloro-2-methylisothiazolone and free from 2-mercaptopyridine N-oxide and derivatives thereof.

2. Preservative according to Claim 1, **characterized in that** the isothiazolone is selected from 2-n-octylisothiazolone, 2-methylisothiazolone, benzoisothiazolone, 4,5-dichloro-2-n-octylisothiazolone, 2-n-butylbenzoisothiazolone and mixtures thereof, preferably 2-n-octylisothiazolone.

3. Preservative according to Claim 1 or 2, **characterized in that** the glycol is monoethylene glycol.

4. Preservative according to one of the preceding claims, **characterized in that** the mixture is free from butyl diglycol.

5. Preservative according to one of the preceding claims which comprises
a) 0.25-5% by weight, preferably 0.5-3% by weight, for example about 1.1% by weight, of isothiazolone and
b) 99.75-95% by weight, preferably 99.5-97% by weight, for example about 98.9% by weight, of the condensation product.

6. Preservative which essentially consists of
a) 0.5 to 3% by weight of 2-n-octylisothiazolone and
b) 99.5 to 97% by weight of the condensation product of a mixture of monoethylene glycol, potassium carbonate, urea and paraformaldehyde.

7. Use of the composition according to any one of the preceding claims for preserving technical products.

8. Use according to Claim 7, **characterized in that** the composition is used at a concentration of 0.01 to 1% by weight.

9. Use of a condensation product according to any one of the preceding claims of a mixture which comprises glycol, urea and formaldehyde for stabilizing isothiazolones different from 5-chloro-2-methylisothiazolone.

10. Technical product which comprises a preservative according to one of Claims 1 to 6.

## Patentansprüche

1. Konservierungsmittel, das
a) Isothiazolon und
b) das Kondensationsprodukt einer Mischung, die 24 bis 44 Gew.-% Glykol, 17 bis 32 Gew.-% Harnstoff und 33 bis 60 Gew.-% Formaldehyd umfasst,
enthält, wobei das Konservierungsmittel frei von 5-Chlor-2-methylisothiazolon und frei von 2-Mercaptopyridin-N-oxid und dessen Derivaten ist.

2. Konservierungsmittel nach Anspruch 1, **dadurch gekennzeichnet, daß** das Isothiazolon ausgewählt ist aus 2-n-Octyl-isothiazolon, 2-Methylisothiazolon, Benzisothiazolon, 4,5-Dichlor-2-n-octylisothiazolon, 2-n-Butylbenzisothialozon und Mischungen davon, bevorzugt 2-n-Octylisothiazolon.

3. Konservierungsmittel nach Anspruch 1 oder 2, **dadurch gekennzeichnet, daß** der Glykol Monoethylenglykol ist.

4. Konservierungsmittel nach einem der vorhergehenden Ansprüche, **dadurch gekennzeichnet, daß** die Mischung frei von Butyldiglykol ist.

5. Konservierungsmittel nach einem der vorhergehenden Ansprüche, das
a) 0,25 bis 5 Gew.-%, vorzugsweise 0,5 bis 3 Gew.-%, wie beispielsweise etwa 1,1 Gew.-%, Isothiazolon und
b) 99,75 bis 95 Gew.-%, vorzugsweise 99,5 bis 97 Gew.-%, beispielsweise etwa 98,9 Gew.-%, des Kondensationsproduktes enthält.

6. Konservierungsmittel, das im Wesentlichen aus
a) 0,5 bis 3 Gew.-% 2-n-Octylisothiazolon und
b) 99,5 bis 97 Gew.-% des Kondensationsprodukts einer Mischung von Monoethylenglykol, Kaliumcarbonat, Harnstoff und Paraformalehyd besteht.

7. Verwendung des Mittels gemäß einem der vorhergehenden Ansprüche zur Konservierung von technischen Produkten.

8. Verwendung nach Anspruch 7, **dadurch gekennzeichnet, daß** die Zusammensetzung in einer Konzentration von 0,01 bis 1 Gew.-% eingesetzt wird.

9. Verwendung eines Kondensationsprodukts gemäß einem der vorhergehenden Ansprüche einer Mischung, die Glykol, Harnstoff und Formaldehyd umfasst, zur Stabilisierung von von 5-Chlor-2-methylisothiazolon verschiedenen Isothiazolonen.

10. Technisches Produkt, das ein Konservierungsmittel gemäß einem der Ansprüche 1 bis 6 umfaßt.

## Revendications

1. Agent conservateur comprenant
a) l'isothiazolone, et
b) le produit de condensation d'un mélange qui comprend de 24 à 44 % en poids de glycol, de 17 à 32 % en poids d'urée et de 33 à 60 % en poids de formaldéhyde,
l'agent conservateur étant dépourvu de 5-chloro-2-méthylisothiazolone et dépourvu de 2-mercaptopyridine-N-oxyde et des dérivés de ceux-ci.

2. Agent conservateur selon la revendication 1, **caractérisé en ce que** l'isothiazolone est choisie parmi la 2-n-octylisothiazolone, la 2-méthylisothiazolone, la benzoisothiazolone, la 4,5-dichloro-2-n-octylisothiazolone, la 2-n-butylbenzoisothiazolone et des mélanges de celles-ci, de préférence la 2-n-octylisothiazolone.

3. Agent conservateur selon la revendication 1 ou 2, **caractérisé en ce que** le glycol est le monoéthylèneglycol.

4. Agent conservateur selon l'une des revendications précédentes, **caractérisé en ce que** le mélange est dépourvu de butyldiglycol.

5. Agent conservateur selon l'une des revendications précédentes, comprenant
a) 0,25-5 % en poids, de préférence 0,5-3 % en poids, par exemple environ 1,1 % en poids, d'isothiazolone, et
b) 99,75-95 % en poids, de préférence 99,5-97 % en poids, par exemple environ 98,9 % en poids, de produit de condensation.

6. Agent conservateur qui est essentiellement constitué de
a) 0,5 à 3 % en poids de 2-n-octylisothiazolone, et
b) 99,5 à 97 % en poids du produit de condensation d'un mélange de monoéthylèneglycol, de carbonate de potassium, d'urée et de paraformaldéhyde.

7. Utilisation de la composition selon l'une quelconque des revendications précédentes, pour la conservation de produits techniques.

8. Utilisation selon la revendication 7, **caractérisée en ce que** la composition est utilisée à une concentration de 0,01 à 1 % en poids.

9. Utilisation d'un produit de condensation selon l'une quelconque des revendications précédentes d'un mélange comprenant le glycol, l'urée et le formaldéhyde pour stabiliser des isothiazolones différentes de la 5-chloro-2-méthylisothiazolone.

10. Produit technique comprenant un agent conservateur selon l'une quelconque des revendications 1 à 6.
